# EUROPÄISCHE PATENTSCHRIFT

(11) **EP 0 691 108 B1**
(45) Veröffentlichungstag und Bekanntmachung des Hinweises auf die Patenterteilung: **04.12.2002**
(21) Anmeldenummer: 95108605.7
(22) Anmeldetag: 06.06.1995
(51) Int. Cl.: A61F 2/06

(54) **In den Körper eines Patienten perkutan implantierbare Endoprothese**
Endoprosthesis percutaneously implantable into the body of a patient
Endoprothèse implantable percutanée dans le corps d'un patient

(30) Priorität: 09.07.1994 DE 4424242
(43) Veröffentlichungstag der Anmeldung: 10.01.1996
(73) Patentinhaber: Strecker, Ernst Peter, Dr.-med.Prof., D-76228 Karlsruhe (DE)
(72) Erfinder: Strecker, Ernst Peter, Dr.-med.Prof., D-76228 Karlsruhe (DE)
(74) Vertreter: Geitz, Heinrich, Dr.-Ing.

(56) Entgegenhaltungen:
- EP-A- 0 221 570
- EP-A- 0 423 916
- EP-A- 0 556 850
- EP-A- 0 645 125
- WO-A-93/13825
- WO-A-95/29646

## Beschreibung

Die Erfindung bezieht sich auf eine in den Körper eines Patienten, insbesondere in röhrenförmige Gefäße oder Organe, mittels eines Katheters perkutan implantierbare Endoprothese, die als länglicher Hohlkörper ausgebildet und - nach lagerichtiger Plazierung bei der Implantation von einem kleinen Lumen beim Einführen auf ein der Gebrauchslage entsprechendes größeres Lumen veränderbar ist.

Perkutan einführbare und im Lumen veränderbare Prothesen sind bekannt. Sie dienen zum Eröffnen oder Erweitern von Gefäßlumen entweder durch mechanisches Aufdehnen mittels bekannter Ballonkatheter von einem kleinen auf ein größeres Lumen oder sie dehnt sich nach vorheriger Zusammendrückung vor der Implantation durch Federkraft, bedingt durch beim Zusammendrücken erzeugte Federvorspannung, von selbst auf.

Eine auf einem Ballonkatheter aufgenommene und durch Dilatation aufweitbare sowie dadurch vom Katheter ablösbare und in einem Gefäß plazierbare Endoprothese ist in der EP-A-0 292 587 beschrieben. Bei dieser Prothese handelt es sich um einen durch Stricken oder Häkeln aus Metall- oder Kunststoffadenmaterial hergestellten Stent in Form eines schlauchartigen Hohlkörpers, bei dem die einzelnen Maschen aus locker ineinandergreifenden Schlingen bestehen. Beim Aufweiten infolge Dilatation des Ballons des Katheters erfahren die ineinandergreifenden Schlingen plastische Verformungen und demgemäß verharrt eine aufgedehnte Prothese in ihrer Aufweitlage.

Selbstaufdehnende Stents sind beispielsweise in der EP-A-0 183 372, der US-A-4 732 152 und der DE-A-41 37 857 vorbeschrieben. Diese Prothesen werden vor der Implantation gegen ihnen eigene Federrückstellkräfte auf einen reduzierten Querschnitt zusammengedrückt, im zusammengedrückten Zustand in den Körper eines Patienten eingeführt und federn nach lagerichtiger Plazierung infolge Wegnahme der Rückhaltekraft im jeweiligen Gefäß oder Körperhohlraum auf und werden dadurch fixiert.

Bei der in der EP-A-0 183 372 beschriebenen Endoprothese handelt es sich darum, daß diese zum Zwecke der Implantation auf einen reduzierten Querschnitt zusammengedrückt und dann in diesem zusammengedrückten Zustand mittels eines Schiebers durch einen zuvor in ein Gefäß eingebrachten Katheter hindurch bis zur lagerichtigen Positionierung im Gefäß vorgeschoben wird. Dieses Vorschieben der Prothese durch den Katheter erfordert einen erheblichen Kraftaufwand, weil der Verschiebung große Reibungskräfte entgegenwirken.

In der US-A-4 732 152 ist eine gewebte und federnd ausgebildete Endoprothese vorgeschrieben, die im zusammengedrückten Zustand durch eine doppelte, am distalen Ende verschlossene Hülle zusammengehalten wird. Diese Hülle wird, wie beim Abstreifen eines Strumpfs vom Fuß eines Trägers, von der zusammengefalteten Prothese zurückgezogen. Zur Vermeidung der dabei auftretenden Reibung kann zwischen die beiden Hüllenblätter Flüssigkeit eingefüllt werden. Das wegen der Reduzierung der Reibungswiderstände zunächst elegant erscheinende System ist jedoch sehr umständlich in der Handhabung.

Aus der DE-A-41 37 857 ist eine als Hohlkörper ausgebildete Prothese vorbekannt, die gegen die Wirkung rückstellender Federkräfte auf einen gegenüber einer aufgeweiteten Gerbrauchslage reduzierten Querschnitt zusammengedrückt und in dieser Lage mittels einer aufziehbaren Umhüllung gehalten ist. Nach dem Aufziehen der Umhüllung weitet sich die Prothese selbsttätig auf einen der Gebrauchslage entsprechenden Querschnitt auf. Die Umhüllung, bei der es sich um ein Maschenwerk etwa in Form einer Umhäkelung handeln kann, reicht über die gesamte Länge der Prothese und besteht aus wenigstens einem durchlaufenden Faden und einer Aufziehleine. Die durch die Umhüllung in radial zusammengedrückter Lage gehaltene Prothese kann vorschiebbar etwa auf einem Führungdraht oder auch axialfest auf dem Ende einer Sonde oder eines Katheters aufgenommen sein.

Bekannt ist schließlich auch schon eine - druckschriftlich nicht belegbare - Endoprothese aus einer Memory-Legierung, bei der es sich um einen länglichen Hohlkörper mit einem vielfach durchbrochenen und in der Art von Streckmetall ausgebildeten Mantel handelt.

Von einem kleineren Einführlumen dehnt sich diese Endoprothese selbsttätig auf ein größeres Lumen auf. Diese Endoprothese ist jedoch wenig flexibel und impliziert jedenfalls bei der Implantation über ein Gelenk die Gefahr von Ermüdungsbrüchen. Die den Mantel bildenden Streben sind in den jeweiligen Eckpunkten nicht alle miteinander verbunden und können sich daher im implantierten Zustand von einer Gefäßwand lösen und in diese hineinragen.

Die EP-A-0 423 916 und die EP-A-0 645 125 (letztere Stand der Technik nach Art. 54(3) EPü) betreffen jeweils einen selbstaufdehnenden Stent, der als ein aus elastischen Filamenten hergestelltes schlauchförmiges Geflecht ausgebildet ist. Dieses weist die Struktur eines Maschendrahtzauns mit Vielecke bildenden Maschen auf. Dabei umgreifen im Falle des aus der EP-A-0 423 916 vorbekannten Stents die Filamente einander in der Form von geschlossene Schleifen ausbildenden Ösen. Ein Zusammenschieben der Maschen dieses Stents ist folglich nicht möglich. Demgegenüber umgreifen die Filamente der Maschen des aus der EP-A-0 645 125 hervorgehenden Stents einander lose. Dabei sind die Enden der Filamente in Längsrichtung des Stents mit den Maschen verwoben, um eine Deformation dieses Stents insbesondere in dessen Längsrichtung zu behindern.

Aus der WO 95 (Stand der Technik nach Art. 54(3) EPü) 29646 ist eine als Stent ausgebildete, in dem Körper eines Patienten mittels eines Katheters implantierbare Endoprothese in der Form eines aus elastischen Filamenten hergestellten schlauchförmigen, Maschen aufweisenden Geflechts bekannt. Dabei können die Maschenverbindungsbereiche zwei gegeneinander verdrillte Filamente aufweisen, die Filamente können sich jedoch auch nur lose umgreifen, ohne miteinander verdrillt zu sein.

Die der Erfindung zugrundeliegende Aufgabe besteht in der Schaffung einer Endoprothese, die im Vergleich zu den vorbekannten Stents nach dem Stande der Technik flexibler ist und ein besseres Beharrungsvermögen im aufgeweiteten Zustand besitzt.

Gelöst ist diese Aufgabe gemäß einem ersten Lösungsgedanken durch die Merkmale des Anspruches 1. Dabei handelt es sich bei der Endoprothese um ein aus elastischen Filamenten hergestelltes schlauchförmiges Geflecht, das die Struktur eines Maschendrahtzauns mit Vielecke bildenden Maschen aufweist, bei dem die Filamente jeweils in Richtung der Prothesenlängsachse aufeinanderfolgender Maschen einander in deren Eckpunkten lose umgreifen, und wobei die Filamente in den Eckpunkten der Maschen mit in der Strecklage der Prothese formschlüssig ineinandergreifenden Verrastungen versehen sind, wobei die Filamente jeweils in Richtung der Protheselängsachse aufeinanderfolgender Maschen einander derart lose umgreifen, daß die Prothese gegenüber ihrer maximalen Länge mit in den Eckpunkten der Maschen aneinander anliegenden Filamenten durch Zusammenschieben der Maschen verkürzbar ist, und daß es sich bei den Verrastungen um durch Zusammenpressen der in der Strecklage der Prothese in den Eckpunkten einander kreuzenden Filamente auf etwa das Dickenmaß eines Filamentes gebildete und in der Strecklage der Prothese ineinandergreifende Einsenkungen handelt, die in den Verrastungslagen formschlüssig ineinandergreifen und in jedem Eckpunkt dann eine formschlüssige Verrastung vermitteln. Derartige Verrastungen vermitteln ein besonders ausgeprägtes Beharrungsvermögen der Prothese in ihrer Aufweitlage.

Eine so gestaltete Endoprothese, die selbstaufdehnend ausgebildet oder mittels eines Ballonkatheters aufweitbar sein kann, ist in einfacher Weise den jeweiligen Bedürfnissen des Anwendungsfalles anpaßbar und stellt ein flexibles Gebilde dar, das auch in Gelenkbereichen unproblematisch implantierbar ist und dann ohne Behinderung des Probanden Gelenkbewegungen zu folgen vermag.

Insbesondere hat sich gezeigt, daß eine derartige Endoprothese ein gegenüber dem Stand der Technik verbessertes Verhältnis zwischen kleinem Radius und großem Radius in der Aufweitlage besitzt. Dies bedeutet, daß der beim Implantieren einzusetzende Einführkatheter kleiner als bei vorbekannten Prothesen gehalten werden kann.

Diese Endoprothese ist somit innerhalb vorgegebener Grenzen längenveränderbar, wobei im Falle maximaler Axialerstreckung die einander umgreifenden Filamente in den Eckpunkten der Maschen aneinander anliegen, hingegen im Falle einer Verkürzung einander mit mehr oder weniger großem Axialspiel umgreifen. Der besondere Vorteil einer derartigen Prothesenausbildung besteht darin, daß jedenfalls beim Aufweiten keinerlei Verkürzung auftritt, weil das beim Aufweiten in radialer Richtung benötigte Material nicht durch Längenverkürzung zur Verfügung gestellt werden muß, sondern durch die axial nicht aneinanderliegenden Filamente der einzelnen Maschen verfügbar ist.

Als besonders zweckmäßig hat sich erwiesen, wenn das die Endoprothese bildende schlauchförmige Geflecht aus wenigstens einem durchlaufenden und unter Maschenbildung rundgeflochtenen Filament besteht. Alternativ dazu kann aber auch die Endoprothese aus einem ursprünglich flachliegenden und zu einer Schlauchform aufgerollten Geflecht hergestellt sein, wobei zwei Längskanten des Geflechts dann mittels einer Längsnaht miteinander verbunden sind.

Gemäß einem alternativen Erfindungsgedanken ist die Aufgabe durch die Merkmale des Anspruchs 4 gelöst. Dabei handelt es sich bei der Endoprothese um ein aus elastischen Filamenten hergestelltes schlauchförmiges Geflecht, das die Struktur eines Maschendrahtzauns mit Vielecke bildenden Maschen besitzt, und daß die Maschen Verbindungsbereiche mit zwei gegeneinander verdrillten Filamenten aufweisen, wobei in den Verbindungsbereichen von in Umfangsrichtung benachbarten Maschen die Filamente jeweils mit wenigstens einer Windung gegeneinander verdrillt sind, und daß die Filamente durch Zusammenpressen in den gegeneinander verdrillten Verbindungsbereichen mit in der Aufweitlage formschlüssig ineinandergreifenden Einsenkungen versehen sind, und wobei unter axialen Zugkräften die durch die Verdrillungen gebildeten Verrastungen außer Eingriff gelangen können und dabei die Prothese bei Verlust ihrer radialen Tragfähigkeit eine Längenausdehnung sowie eine Querschnittsänderung zu einem kleinen Lumen erfährt.

Eine Endoprothese mit den vorstehenden Lösungsmerkmalen zeichnet sich durch ein besonders ausgeprägtes Beharrungsvermögen in der Aufweitlage ohne Beeinträchtigung der Flexibilität des schlauchförmigen Geflechtes aus. Eine so gestaltete Endoprothese kann mit kleinem Querschnitt und entsprechender Längenausdehnung unproblematisch beispielsweise in einem Blutgefäß mittels eines geeigneten Katheters vorgeschoben und dann bei vorherbestimmter Verkürzung durch radiales Aufweiten in ihre Gebrauchslage gebracht werden.

Zweckmäßigerweise erstrecken sich bei einer derartigen Endoprothese die Verbindungsbereiche mit zwei gegeneinander verdrillten Filamenten in Prothesenlängsrichtung. Gemäß einer sinnvollen Weiterbildung können die Verbindungsbereiche aber auch längs - gedachter - schraubengangförmiger Linien eines von der Prothese aufgespannten - ebenfalls gedachten - Zylindermantels verlaufen und eine Helixstruktur bilden. Dabei kann die Anordnung auch so getroffen sein, daß die Helixstruktur durch Richtungsänderung der Verbindungsabschnitte aufeinanderfolgender Maschen derart unterbrochen ist, daß bei einem Teil der Maschen die sich zwischen diesen erstreckenden Verbindungsbereiche im Winkel zu den Verbindungsbereichen zwischen anderen Maschen verlaufen, was zu einer Struktur wie bei "Fischschuppen" führt.

Bei dieser Endoprothese können in Umfangsrichtung benachbarte Maschen jeweils von zwei Filamenten und in axialer Richtung aufeinanderfolgende sowie jeweils über einen Verbindungsbereich miteinander verbundene Maschen aus jeweils denselben beiden Filamenten gebildet sein. Alternativ dazu können aber auch in Umfangsrichtung aneinandergrenzende Maschen jeweils von zwei Filamenten und die jeweils axial und in Umfangsrichtung benachbarten Maschen von einem dieser Filamente und je einem anderen Filament gebildet sein, wobei in axialer Richtung von Masche zu Masche fortschreitend die Filamente an der Bildung in Umfangsrichtung jeweils benachbarter Maschen beteiligt sind und sich nach und nach treppenstufenartig um den Stent herumerstrecken. Eine derartige Stentausbildung hat sich als einfach herstellbar und hinsichtlich ihres Beharrungsvermögens in der Aufweitlage vorteilhaft erwiesen.

Eine abermalige Weiterbildung der alternativen Aufgabenlösung sieht vor, daß sich jeweils ein Filament in den Verbindungsbereichen unverdrillt in Prothesenlängsrichtung erstreckt und daß um dieses Filament das andere Filament spiralförmig herumgewunden ist. Eine solche Ausbildung ermöglicht ein begrenztes Entlanggleiten des einen Filaments auf dem geradlinigen Abschnitt des anderen Filaments sowie angesichts der spiralförmigen Windungen auch eine Stauchung und Streckung des einen Filaments. Dies ermöglicht eine verbesserte Anpassung einer so ausgebildeten Endoprothese in Kurvenbereichen.

Eine nochmalige Weiterbildung kann auch durch eine Verrastung mit wenigstens einer Haltestange gekennzeichnet sein, die sich in Prothesenlängsrichtung erstreckt und mit ihrem einen Ende fest im Geflecht verankert sowie am anderen Ende mit einem Haken zum Einrasten in eine Masche versehen ist. Beim radialen Aufweiten einer mit einer derartigen Verrastung ausgerüsteten Endoprothese gleitet das mit dem Haken vesehene Ende der Haltestange beim radialen Aufweiten und der dabei einhergehenden Verkürzung des Geflechts über die Maschen und hinterfaßt die Filamente einer Masche mit der Folge, daß nach dem Aufweiten das schlauchförmige Geflecht an einer radialen Zusammendrückung dadurch gehindert wird, daß eine mit einer radialen Verkleinerung einhergehende Längenvergrößerung nicht mehr möglich ist.

Zweckmäßigerweise kann die Haltestange außenseitig an dem schlauchförmigen Geflecht sich entlang erstrecken und mithin im implantierten Zustand, zwischen einer Gefäßwand und dem schlauchförmigen Geflecht aufgenommen sein. Dadurch ist sichergestellt, daß unter keinen Umständen eine Beeinträchtigung des Lumens eintritt. Die Haltestange kann sich aber auch in der Art eines Kettfadens durch das Maschenwerk des Geflechts hindurcherstrecken, wodurch ebenfalls das Lumen freigehalten wird.

Zweckmäßigerweise kann bei einer Weiterbildung der zweiten Lösungsvariante vorgesehen sein, daß zur Längenbegrenzung der als Stent ausgebildeten Endoprothese das schlauchförmige Geflecht mit sich in Stentlängsrichtung erstreckenden Kettfäden ausgerüstet ist, die zumindest im Bereich der Prothesenenden mit den die Maschen bildenden Filamenten verbunden sein können. Diese Kettfäden können aus Textilfilamenten bestehen, und in solcher Weise dicht benachbart angeordnet sein, daß sie einen den Stent einschließenden Mantel bilden.

Gemäß einer Weiterbildung des zuletztgenannten Merkmals können die Kettfäden aus biologisch abbaubarem Material bestehen und/oder als Medikamentendepots ausgebildet sein und bei ihrem Abbau Medikamente freigeben.

Eine abermalige Weiterbildung sieht vor, daß die Kettfäden aus dehnbarem Material bestehen, wie texturierten Textilfäden. Die Kettfäden können aber auch aus unelastischem Material hoher Dichte und Protonenzahl bestehen, was sich besonders vorteilhaft bei der Implantation unter Röntgenkontrolle erwiesen hat, weil derartiges Material in hohem Maße Röntgenstrahlen absorbiert und daher im Röntgenbild gut sichtbar ist.

Zweckmäßigerweise sollten dabei an den Stirnenden des schlauchförmigen Geflechts auch die Enden der Filamente abgebogen und mit dem Geflecht verbunden sein, um Verletzungen während der Implantation oder am Implantationsort wirksam zu verhindern. So können die abgebogenen Enden der Filamente an den Stirnenden des schlauchförmigen Geflechts eingeflochten, verklebt, verlötet oder verschweißt, aber auch als Ösen ausgebildet sein.

Eine ebenfalls wichtige Weiterbildung der beiden Lösungsvarianten sieht vor, daß die die Maschen des schlauchförmigen Geflechts bildenden Filamente auf der zum Lumen hinweisenden Seite abgerundet oder abgeflacht ausgebildet sind, um günstige Strömungseigenschaften an der Innenwand eines eine derartige Endoprothese aufnehmenden Gefäßes zu erzielen.

Schließlich hat sich als vorteilhaft erwiesen, wenn die das Geflecht bildenden Filamente bei beiden Lösungsvarianten aus superelastischem Material oder einem Memorymaterial bestehen, etwa aus Nitinol.

Beide Lösungsvarianten können im Rahmen der vorliegenden Erfindung als ballonaufdehnbare oder auch als selbstaufdehnbare Endoprothesen ausgebildet sein.

Anhand der beigefügten Zeichnungen sollen nachstehend verschiedene Ausführungsmöglichkeiten der erfindungsgemäßen Endoprothese erläutert werden. In schematischen Ansichten zeigen:
- Fig. 1: eine als schlauchartiges Geflecht mit der Struktur eines Maschendrahtzauns ausgebildete Endoprothese in einer perspektivischen Ansicht,
- Fig. 2: eine stirnseitige Ansicht der Endoprothese mit Blickrichtung gemäß Pfeil II in Fig. 1,
- Fig. 3: in einer ausschnittsweisen Abwicklung des Prothesenmantels den Aufbau des Geflechts aus Vielecke bildenden und in den jeweiligen Kreuzungspunkten nicht miteinander verbundenen Filamenten,
- Fig. 4: das Geflecht in einer Ansicht wie in Fig. 3, jedoch bei axial gestauchter Prothese,
- Fig. 5: einen Kreuzungsbereich der Filamente, der mit Verrastungen versehen ist, die bei maximaler Längenausdehnung der Prothese formschlüssig ineiriandergreifen,
- Fig. 6: eine der Schnittlinie VI-VI in Fig. 5 entsprechende Schnittansicht durch einen Kreuzungsbereich,
- Fig. 7: in einer Ansicht wie in Fig. 3 eine weitere Geflechtalternative,
- Fig. 8: in einer vergrößerten Ausschnittansicht aus Fig. 7 die Bildung jeweils in Prothesenlängsrichtung aufeinanderfolgender Maschen aus jeweils zwei Filamenten,
- Fig. 9: in einer Ansicht wie in Fig. 8 eine Maschenbildung, bei der die Filamente nach und nach um die Endoprothese herumgeflochten und an der Bildung jeweils in Längs- und Umfangsrichtung benachbarter Maschen beteiligt sind,
- Fig. 10: alternativ zu Fig. 9 in einer vergrößerten Ausschnittansicht einen Verbindungsbereich, in dem sich das eine Filament geradlinig in Prothesenlängsrichtung erstreckt und um dieses Filament das andere Filament herumgewunden ist,
- Fig. 11: in einer Ansicht wie in Fig. 7 einen Geflechtausschnitt einer nicht aufgedehnten Endoprothese mit einer sich in Prothesenlängsrichtung erstreckenden Haltestange und
- Fig. 12: das Geflecht nach Fig. 11 im aufgeweiteten und mittels der Haltestange in der Aufweitlage verrasteten Zustand,
- Fig. 13: in einer Ansicht wie in Fig. 7 einen Prothesenaufbau mit einen maschendrahtzaunartigen Geflecht, bei dem die Verbindungsabschnitte zwischen benachbarten Maschen einerseits schräg zur Prothesenlängsachse verlaufend sich schraubengangförmig um einen - gedachten - Zylindermantel herumerstrecken und andererseits die Richtung ändern, so daß eine fischschuppenartige Struktur entsteht.

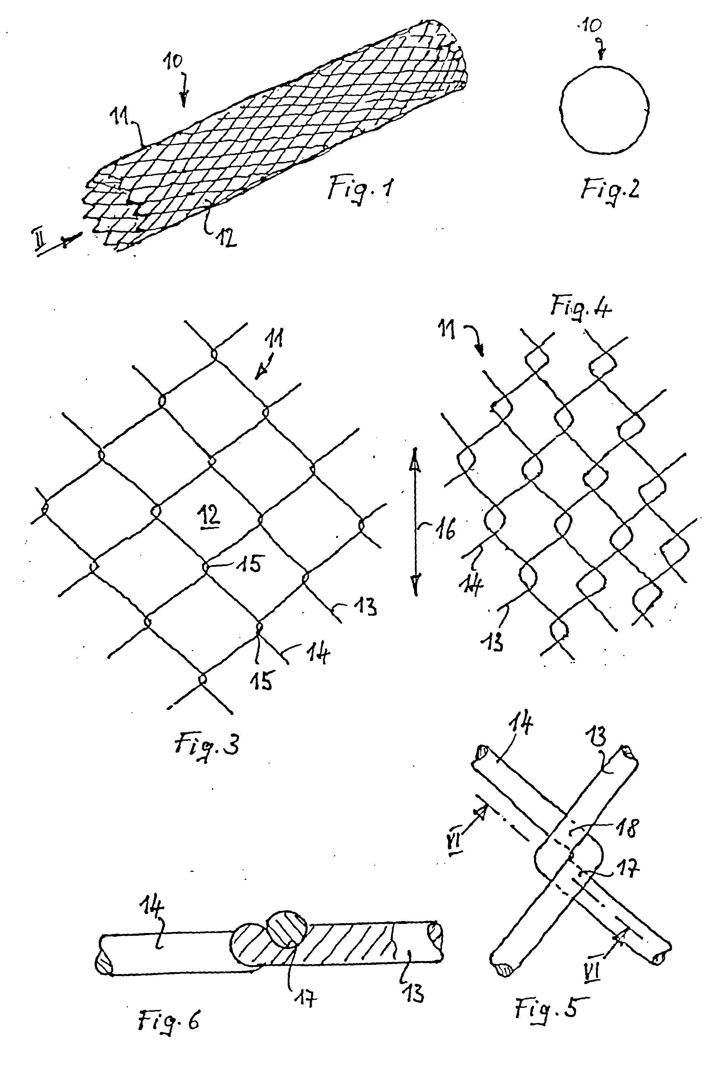

Bei der in den Fig. 1 bis 4 veranschaulichten Endoprothese 10 handelt es sich um ein schlauchförmiges Geflecht 11 mit Vielecke bildenden Maschen 12. Diese Maschen sind von Filamenten 13, 14 gebildet, die einander in den Eckpunkten 15 ohne feste Verbindung miteinander umgreifen, und zwar jeweils die Filamente der in Prothesenlängsrichtung benachbarten Maschen. Insbesondere die in den Fig. 3 und 4 gezeigten Abwicklungen des Mantels der Endoprothese zeigen die maschendrahtzaunartige Struktur des Geflechts 11, wobei der Pfeil 16 die sich quer zur Prothesenlängsachse erstreckende Tragrichtung andeutet. Fig. 3 veranschaulicht das Maschenbild mit in allen Eckpunkten 15 einander anliegenden Filamenten bei maximaler Längenausdehnung der Prothese 10, hingegen Fig. 4 das Maschenbild bei axial gestauchter Prothese, bei dem die Filamente 13, 14 in den Eckpunkten einander mit großem Spiel umgreifen.

Die erfindungsgemäße Prothese 10 kann selbstaufweitend oder ballonaufdehnbar ausgebildet sein, wobei keinerlei axiale Verkürzung beim Aufweiten eintritt, wenn die Prothese im nicht aufgeweiteten Zustand gegenüber ihrer maximalen Längenausdehnung verkürzt ist und etwa ein Maschenbild gemäß Fig. 4 aufweist. Wenn die Filamente 13, 14 aus der in Fig. 3 ersichtlichen Weise bei maximaler Längenausdehnung der Prothese in den Eckpunkten 15 einander ohne Spiel umgreifen, führt das radiale Aufweiten naturgemäß zu einer Längenverkürzung. Sofern eine derartige Prothese vor dem Aufweiten entsprechend dem Maschenbild gemäß Fig. 4 axial gestaucht ist, tritt hingegen keinerlei Längenverkürzung beim Aufweiten ein und mithin ist eine präzise Plazierung am Implantationsort gewährleistet.

Die Fig. 3 und 4 zeigen, daß die Filamente 13, 14 einander in den in Längsrichtung der Prothese aufeinanderfolgenden Eckpunkten 15 der Vielecke bildenden Maschen 12 einander umgreifen. Eine derartige Ausbildung der Endoprothese ist Voraussetzung für deren radiale Tragfähigkeit, die der quer zur Prothesenlängsachse gerichtete Pfeil 16 zwischen den Fig. 3 und 4 andeutet.

Eine gute radiale Tragfähigkeit der Prothese wird dadurch erreicht, daß die die Maschen bildenden Filamente in den Kreuzungspunkten mit formschlüssig ineinandergreifenden Verrastungen versehen sind. Wie die Fig. 5 und 6 zeigen, sind bei maximaler Längenausdehnung der Prothese die einander in den Eckpunkten 15 der Vielecke kreuzenden Filamente 13, 14 quer zur Prothesenlängserstreckung derart ineinandergedrückt, daß jeweils das eine Filament in einer Einsenkung 17, 18 des anderen Filamentes in der Weise formschlüssig aufgenommen ist, daß beide Filamente zusammen auf die Stärke etwa eines Filaments reduziert sind und angesichts des formschlüssigen Eingriffs ineinander sich gegenseitig verhaken und dadurch eine gute radiale Tragfähigkeit der Endoprothese vermitteln.

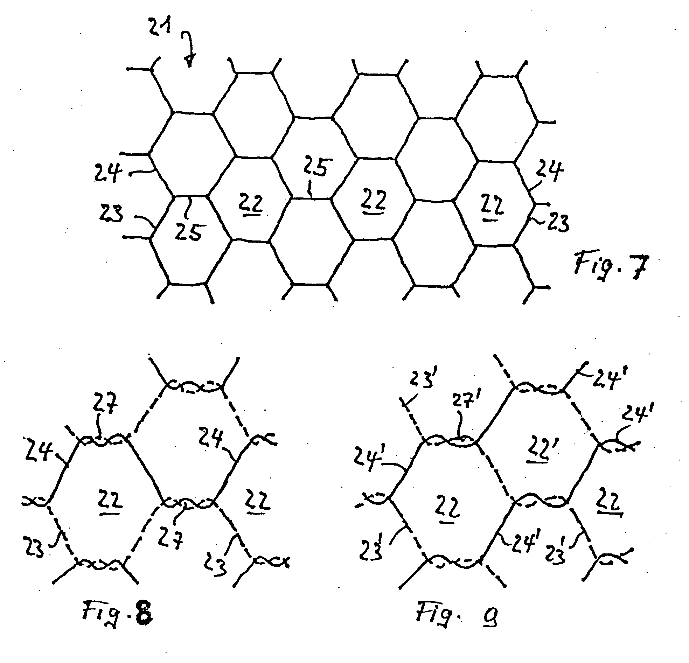

Bei der in Fig. 7 ausschnittsweise dargestellten Abwicklung des Mantels einer Endoprothese handelt es sich um ein Geflecht 21 in der Art eines sogenannten Kaninchendrahtzauns, bei dem die Maschen 22 als Sechsecke ausgebildet und jeweils in Umfangsrichtung und in axialer Richtung durch einen Verbindungsbereich 25 voneinander getrennt sind. In diesen Verbindungsbereichen sind jeweils zwei Filamente 23, 24 miteinander verdrillt. Diese Verdrillungen bilden formschlüssig ineinandergreifende Verrastungen, die in der radialen Aufweitlage einer von diesem Geflecht 21 gebildeten Endoprothese deren Beharrung in der Aufweitlage vermitteln.

Die Verbindungsbereiche 25 zwischen den in Längs- und Umfangsrichtung der Prothese benachbarten Maschen 22 erstrecken sich in Prothesenlängsrichtung und die Verdrillungen der an der jeweiligen Maschenbildung beteiligten Filamente 23, 24 in den Verbindungsbereichen 25 sind so ausgebildet, daß sie bei radial aufgeweiteter Prothese formschlüssig ineinandergreifen und dadurch die Prothese in ihrer Aufweitlage sichern, bei Aufbringung axialer Zugkräfte auf die Prothese aber außer. Eingriff gelangen und dadurch die Reduzierung des Prothesenquerschnittes auf ein vergleichsweise kleines Lumen ermöglichen.

Demgemäß handelt es sich bei dieser Prothese um eine mittels eines Ballonkatheters aufweitbare Endoprothese, die bei entsprechender Längenerstreckung auf dem Ballonabschnitt des Katheters aufgenommen wird und beim radialen Aufweiten nach der Implantation beispielsweise in ein Blutgefäß eine entsprechende axiale Verkürzung bis zum formschlüssigen Einrasten der einander umschlingenden Verdrillungen erfährt.

Bei dem in einer vergrößerten Ausschnittansicht in Fig. 8 veranschaulichten Geflecht 21 sind die in Prothesenlängsrichtung aufeinanderfolgenden Maschen jeweils von den beiden selben Filamenten 23, 24 gebildet. Demgemäß verlaufen die beiden Filamente von ihrem jeweiligen Verbindungsbereich 25 unter einem Winkel von etwa 60° nach außen zu den Verbindungsbereichen zwischen in Umfangsrichtung benachbarten Maschen 22 und von diesen Verbindungsbereichen dann wieder zurück zu einem Verbindungsbereich mit der in Prothesenlängsrichtung folgenden Masche. In Fig. 8 zeigen dies die einerseits in vollen Linien und andererseits gestrichelt dargestellten Filamente 23, 24. Desgleichen sind der Verbindungsbereich 25 mit in der Aufweitlage Verrastungen bildenden Verdrillungen 27 dargestellt.

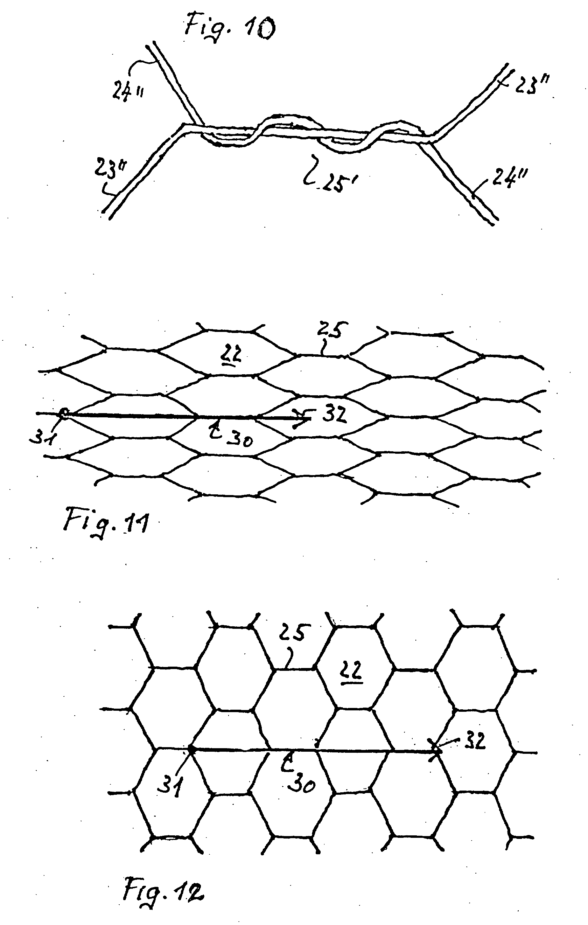

Bei der Ausführungsform gemäß Fig. 9 hingegen sind in Prothesenlängsrichtung aufeinanderfolgende Maschen 22 nicht von den jeweils selben Filamenten 23', 24' gebildet, sondern von Masche zu Masche fortschreitend sind die Filamente an der Bildung in Umfangsrichtung benachbarter Maschen 22' beteiligt. Demgemäß sind die einzelnen Filamente von Masche zu Masche in Prothesenlängerichtung fortschreitend um das schlauchförmige Geflecht herumgeflochten. Dies und die mit Verrastungen bildenden Verdrillungen versehenen Verbindungsbereiche 27' sind gleichfalls durch einerseits in vollen Linien und andererseits gestrichelt dargestellte Filamente veranschaulicht.

Bei der Ausführungsform gemäß Fig. 10 erstreckt sich im Verbindungsbereich 25' das Filament 23'' geradlinig fort und das Filament 24'' ist spiralförmig um das Filament 23'' herumgewunden. Dadurch kann das Filament 24'' begrenzt auf dem geradlinigen Abschnitt des Filaments 23'' gleiten und auch innerhalb vorgegebener Grenzen eine Stauchung oder Streckung erfahren. Diese Endoprothese zeichnet sich somit durch vorzügliche Flexibilität aus und vermittelt auch eine bessere Anpassung in Beugungsbereichen.

Die Fig. 11 und 12 zeigen ein Geflecht wie in Fig. 7, wobei Fig. 11 den nicht aufgeweiteten Zustand, hingegen Fig. 12 den aufgeweiteten Zustand veranschaulicht. im Gegensatz zu der in Fig. 7 veranschaulichten Ausführungsform sind jedoch bei der Ausführungsform nach den Fig. 11 und 12 besondere Rastmittel in Form wenigstens einer sich in Prothesenlängsrichtung erstreckenden Haltestange 30 vorgesehen, die mit ihrem einen Ende bei 31 mit dem das Geflecht bildenden Maschenwerk fest verbunden ist, während das andere Ende der Haltestange einen Haken 32 aufweist. Diese Haltestange kann sich außenseitig an dem die Endoprothese bildenden Geflecht entlang erstrecken oder auch in der Art wie ein Kettfaden durch das Maschenwerk hindurch.

Beim radialen Aufweiten aus dem in Fig. 11 veranschaulichten Zustand mit kleinem Lumen gleitet der Haken 32 angesichts der korrespondierend zum Aufweiten eintretenden Längenverkürzung über die Maschen 22 hinweg und hintergreift in der aus Fig. 12 ersichtlichen Weise die Filamente einer Masche, wodurch nach dem Aufweiten eine erneute Längenvergrößerung und damit eine Querschnittsreduzierung wirksam verhindert wird.

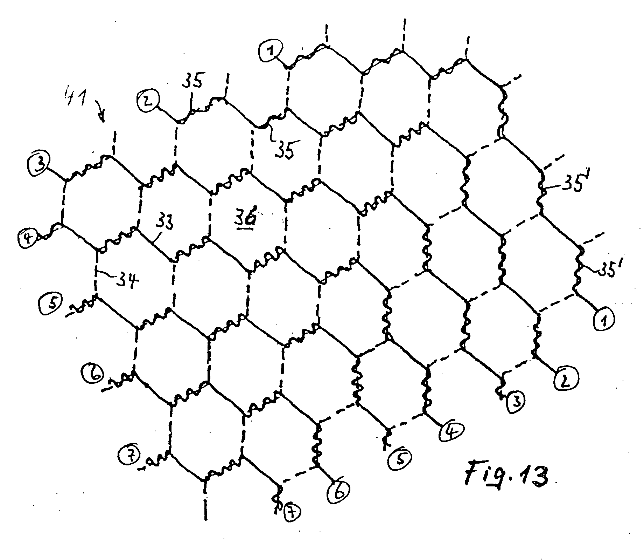

Während bei der Endoprothese nach Fig. 7 die aus jeweils zwei miteinander verdrillten Filamenten 23, 24 bestehenden Verbindungsbereiche 25 sich in Prothesenlängsrichtung erstrecken, sind bei der Ausführungsform 41 gemäß Fig. 13 die miteinander verdrillten Verbindungsbereiche 35, 35' zum Teil schräg zu einer Längsschse der Prothese verlaufend angeordnet und erstrecken sich demgemäß schraubengangförmig um einen von der Prothese gebildeten - gedachten - Zylindermantel herum, und ein Teil der Verbindungsbereiche 35, 35' ist im Winkel zu den erstgenannten Verbindungsbereichen ausgerichtet. Angesichts dieser Richtungsänderung erfährt die Helixstruktur eine Änderung und die als Sechsecke ausgebildeten Maschen 36 bilden eine Struktur in der Art wie Fischschuppen.

In Fig. 13 sind die jeweils einen Filamente 33 in ausgezogenen Linien dargestellt, hingegen die anderen Filamente 34 gestrichelt. Den Verlauf der in der Zeichnung in ausgezogenen Linien dargestellten Filamente 33 verdeutlichen dabei die diesen Filamenten zugeordneten Ziff. 1 bis 7. Die Filamente 33 bilden jeweils mit einem Filament 34 mehrere schräg zur Prothesenlängsachse verlaufende Verbindungsbereiche 35 zwischen benachbarten Maschen 36 und dann im Winkel dazu und etwa rechtwinklig zur Prothesenlängsachse verlaufende Verbindungsbereiche 35', die sich somit in Umfangsrichtung auf dem - gedachten - Zylindermantel der Prothese erstrecken.

## Patentansprüche

1. In den Körper eines Patienten, insbesondere in röhrenförmige Gefäße oder Organe, mittels eines Katheters perkutan implantierbare Endoprothese (10), die als länglicher Hohlkörper ausgebildet und - nach lagerichtiger Plazierung bei der Implantation - von einem kleinen Lumen beim Einführen auf ein der Gebrauchslage entsprechendes größeres Lumen veränderbar ist,
wobei es sich bei der Endoprothese (10) um ein aus elastischen Filamenten (13, 14) hergestelltes schlauchförmiges Geflecht (11) handelt, das die Struktur eines Maschendrahtzauns mit Vielecke bildenden Maschen (12) aufweist, bei dem die Filamente jeweils in Richtung der Prothesenlängsachse aufeinanderfolgender Maschen einander in deren Eckpunkten (15) lose umgreifen, und wobei die Filamente (13, 14) in den Eckpunkten (15) der Maschen (12) mit in der Strecklage der Prothese formschlüssig ineinandergreifenden Verrastungen (17, 18) versehen sind,
**dadurch gekennzeichnet,**
**daß** die Filamente jeweils in Richtung des Prothesenlängsachse aufeinanderfolgender Maschen einander derart lose umgreifen, daß die Prothese gegenüber ihrer maximalen Länge mit in den Eckpunkten (15) der Maschen aneinander anliegenden Filamenten durch Zusammenschieben der Maschen verkürzbar ist, und daß es sich bei den Verrastungen um durch Zusammenpressen der in der Strecklage der Prothese in den Eckpunkten (15) einander kreuzenden Filamente (13, 14) auf etwa das Dickenmaß eines Filamentes gebildete und in der Strecklage der Prothese ineinandergreifende Einsenkungen (17, 18) handelt.

2. Endoprothese nach Anspruch 1, **dadurch gekennzeichnet, daß** das schlauchförmige Geflecht (11) aus wenigstens einem durchlaufenden und unter Maschenbildung rundgeflochtenen Filament (13, 14) besteht.

3. Endoprothese nach Anspruch 1, **dadurch gekennzeichnet, daß** ein ursprünglich flachliegendes Geflecht zu einer Schlauchform aufgerollt ist und danach zwei Längskanten des Geflechts mittels einer Längsnaht miteinander verbunden sind.

4. In den Körper eines Patienten, insbesondere in röhrenförmige Gefäße oder Organe, mittels eines Katheters perkutan implantierbare Endoprothese (10), die als länglicher Hohlkörper ausgebildet und - nach lagerichtiger Plazierung bei der Implantation - von einem kleinen Lumen beim Einführen auf ein der Gebrauchslage entsprechendes größeres Lumen veränderbar ist, wobei es sich bei der Endoprothese (10) um ein aus elastischen Filamenten (23, 24; 23', 24'; 33, 34) hergestelltes schlauchförmiges Geflecht (21, 41) handelt, das die Struktur eines Maschendrahtzauns mit Vielecke bildenden Maschen (22, 22', 36) besitzt, und daß die Maschen Verbindungsbereiche (25, 35, 35') mit zwei gegeneinander verdrillten Filamenten aufweisen,
**dadurch gekennzeichnet,**
**daß** in den Verbindungsbereichen (25) von in Umfangsrichtung benachbarten Maschen die Filamente (23, 24; 23', 24') jeweils mit wenigstens einer Windung gegeneinander verdrillt sind, und daß die Filamente (23, 24; 23', 24')
durch Zusammenpressen in den gegeneinander verdrillten Verbindungsbereichen mit in der Aufweitlage formschlüssig ineinandergreifenden Einsenkungen versehen sind, und wobei unter axialen Zugkräften die durch die Verdrillungen (27, 27') gebildeten Verrastungen außer Eingriff gelangen können und dabei die Prothese bei Verlust ihrer radialen Tragfähigkeit eine Längenvergrößerung sowie eine Querschnittsänderung zu einem kleineren Lumen erfährt.

5. Endoprothese nach Anspruch 4, **dadurch gekennzeichnet, daß** die Verbindungsbereiche (25) mit zwei gegeneinander verdrillten Filamenten (23, 24; 23', 24') sich in Prothesenlängsrichtung erstrecken.

6. Endoprothese nach Anspruch 4, **dadurch gekennzeichnet, daß** die Verbindungsbereiche (35, 35') mit zwei gegeneinander verdrillten Filamenten (33, 34) sich längs - gedachter - schraubengangförmiger Linien eines von der Prothese aufgespannten - gedachten - Zylindermantels erstrecken und eine Helixstruktur bilden.

7. Endoprothese nach Anspruch 6, **dadurch gekennzeichnet, daß** die Helixstruktur durch Richtungsänderung der Verbindungsbereiche (35, 35') aufeinanderfolgender Maschen (32) derart unterbrochen ist, daß bei einem Teil der Maschen die sich zwischen diesen erstreckenden Verbindungsbereiche (35') im Winkel zu den Verbindungsbereichen (35) zwischen anderen Maschen verlaufen.

8. Endoprothese nach einem der Ansprüche 4 bis 6, **dadurch gekennzeichnet, daß** in Umfangsrichtung benachbarte Maschen (22, 36) jeweils von zwei Filamenten (23, 24; 33, 34) und in axialer Richtung aufeinanderfolgende sowie jeweils über einen Verbindungsbereich (25, 35) miteinander verbundene Maschen (22, 36) aus denselben Filamenten gebildet sind.

9. Endoprothese nach einem der Ansprüche 4 bis 6, **dadurch gekennzeichnet, daß** in Umfangsrichtung aneinandergrenzende Maschen (22, 22') jeweils von zwei Filamenten (23, 23') und die jeweils axial und in Umfangsrichtung benachbarten Maschen von einem dieser Filamente und je einem anderen Filament gebildet sind, wobei in axialer Richtung von Masche zu Masche die Filamente an der Bildung der in Umfangsrichtung jeweils benachbarten Maschen beteiligt sind und sich nach und nach treppenstufenartig um die als Stent ausgebildete Endprothese herumerstrecken.

10. Endoprothese nach Anspruch 4, **dadurch gekennzeichnet, daß** sich jeweils ein Filament (23'') in den Verbindungsbereichen (25') unverdrillt in Prothesenlängsrichtung erstreckt und daß um dieses Filament (23'') das andere Filament (24'') spiralförmig herumgewunden ist.

11. Endoprothese nach einem der Ansprüche 4 bis 10, **gekennzeichnet durch** eine Verrastung mit wenigstens einer Haltestange (30), die sich in Prothesenlängsrichtung erstreckt und mit ihrem einen Ende fest im Geflecht verankert sowie am anderen Ende mit einem Haken (32) zum Einrasten in eine Masche versehen ist.

12. Endoprothese nach Anspruch 11, **dadurch gekennzeichnet, daß** sich die Haltestange (30) außenseitig an dem schlauchförmigen Geflecht entlang erstreckt.

13. Endoprothese nach Anspruch 11, **dadurch gekennzeichnet, daß** die Haltestange (30) sich in der Art eines Kettfadens durch das Maschenwerk des Geflechts hindurcherstreckt.

14. Endoprothese nach einem der Ansprüche 4 bis 13, **dadurch gekennzeichnet, daß** zur Längenbegrenzung der als Stent ausgebildeten Endoprothese das schlauchförmige Geflecht mit sich in Stentlängsrichtung erstreckenden Kettfäden ausgerüstet ist.

15. Endoprothese nach Anspruch 14, **dadurch gekennzeichnet, daß** die Kettfäden zumindest im Bereich der Prothesenenden mit den die Maschen bildenden Filamenten verbunden sind.

16. Endoprothese nach Anspruch 14 oder 15, **dadurch gekennzeichnet, daß** die Kettfäden aus Textilfilamenten bestehen und in solcher Weise dicht benachbart angeordnet sind, daß sie einen den Stent einschließenden Mantel bilden.

17. Endoprothese nach einem der Ansprüche 14 bis 16, **dadurch gekennzeichnet, daß** die Kettfäden aus biologisch abbaubarem Material bestehen.

18. Endoprothese nach Anspruch 17, **dadurch gekennzeichnet, daß** die Kettfäden als Medikamentendepots ausgebildet sind und bei ihrem Abbau Medikamente freigeben.

19. Endoprothese nach einem der Ansprüche 14 bis 16, **dadurch gekennzeichnet, daß** die Kettfäden aus dehnbarem Material bestehen, wie texturierten Textilfäden.

20. Endoprothese nach Anspruch 14 oder 15, **dadurch gekennzeichnet, daß** die Kettfäden aus unelastischem Material hoher Dichte und Protonenzahl bestehen.

21. Endoptrohese nach einem der Ansprüche 1 bis 20, **dadurch gekennzeichnet, daß** an den Stirnenden des schlauchförmigen Geflechts die Enden der Filamente abgebogen und mit dem Geflecht verbunden sind, etwa eingeflochten, verklebt, verlötet oder verschweißt.

22. Endoprothese nach Anspruch 21, **dadurch gekennzeichnet, daß** die Enden der Filamente an den Stirnenden des schlauchförmigen Geflechts als Ösen ausgebildet sind.

23. Endoprothese nach einem der Ansprüche 1 bis 22, **dadurch gekennzeichnet, daß** die die Maschen des schlauchförmigen Geflechts bildenden Filamente auf der zum Lumen hinweisenden Seite abgerundet bzw. abgeflacht ausgebildet sind.

24. Endoprothese nach einem der Ansprüche 1 bis 23, **dadurch gekennzeichnet, daß** die das Geflecht bildenden Filamente aus superelastischem Material bestehen.

25. Endoprothese nach einem der Ansprüche 1 bis 24, **dadurch gekennzeichnet, daß** die das Geflecht bildenden Filamente aus Memory-Material bestehen, etwa aus Nitinol.

## Claims

1. Endoprosthesis (10) which is percutaneously implantable in the body of a patient, in particular in tubular vessels or organs, by means of a catheter and which is formed as an elongate hollow body and - after positionally correct placing during the implantation-variable from a small lumen during introduction to a larger lumen corresponding with the position of use, wherein the endoprosthesis (10) is a hoselike mesh (11) which is made of elastic filaments (13, 14) and has the structure of a wire netting with stitch loops (12) forming polygons, in which the filaments of respective stitch loops following one another in the direction of the prosthesis longitudinal axis loosely engage around one another at the corner points (15) of the stitch loops, and wherein the filaments (13, 14) are provided at the corner points (15) of the stitch loops (12) with detents (17, 18) mechanically positively interengaging in the stretched position of the prosthesis, **characterised in that** the filaments of respective stitch loops following one another in the direction of the prosthesis longitudinal axis loosely engage around one another in such a manner that by pushing together the stitch loops the prosthesis is shortenable relative to its maximum length with filaments bearing against one another at the corner points (15) of the stitch loops, and that the detents are hollows (17, 18) which are formed by pressing together the filaments (13, 14), which in the stretched position of the prosthesis intersect at the corner points (15), to approximately the thickness dimension of one filament and which engage in one another in the stretched position of the prosthesis.

2. Endoprosthesis according to claim 1, **characterised in that** the hoselike mesh (11) consists of at least one continuous filament (13, 14) circularly woven while forming stitch loops.

3. Endoprosthesis according to claim 1, **characterised in that** a mesh originally lying flat is rolled into a hose form and thereafter two longitudinal edges of the mesh are connected together by means of a longitudinal seam.

4. Endoprosthesis (10) which is percutaneously implantable into the body of a patient, in particular in tubular vessels or organs, by means of a catheter and which is formed as an elongate body and - after positionally correct placing during the implantation - is variable from a small lumen during the introduction to a larger lumen corresponding with the position of use, wherein the endoprosthesis (10) is a hoselike mesh (21, 41) which is made of elastic filaments (23, 24; 23', 24'; 33, 34) and which has the structure of a wire netting with stitch loops (22, 22', 36) forming polygons, and that the stitch loops have connecting regions (25, 35, 35', with two filaments twisted relative to one another, **characterised in that** in the connecting regions (25) of stitch loops adjacent in circumferential direction the filaments (23, 24; 23', 24') are each twisted relative to one another by at least one turn and that the filaments (23, 24; 23', 24') are provided, by pressing together, in the mutually twisted connecting regions with hollows mechanically positively engaging in one another in the expanded position, and wherein under axial tension forces the detents formed by the twistings (27, 27') can come out of engagement and **in that** case the prosthesis experiences, with loss of its radial load-bearing capability, an enlargement in length as well as a change in cross-section to a smaller lumen.

5. Endoprosthesis according to claim 4, **characterised in that** the connecting regions (25) extend in the prosthesis longitudinal direction by two filaments (23, 24; 23', 24') twisted relative to one another.

6. Endoprosthesis according to claim 4, **characterised in that** the connecting regions (35, 35') extend by two mutually twisted filaments (33, 34) along notional helical lines of a notional cylindrical circumference spanned by the prosthesis and form a helix structure.

7. Endoprosthesis according to claim 6, **characterised in that** the helix structure is interrupted by directional change of the connecting regions (35, 35') of successive stitch loops (32) in such a manner that in the case of a part of the stitch loops the connecting regions (35') extending therebetween run at an angle to the connecting regions (35) between other stitch loops.

8. Endoprosthesis according to one of claims 4 to 6, **characterised in that** stitch loops (22, 36) adjacent in circumferential direction are each formed by two filaments (23, 24; 33, 34) and stitch loops (22, 36) following one another in axial direction as well as interconnected each time by way of a connecting region (25, 35) are formed from the same filaments.

9. Endoprosthesis according to one of claims 4 to 6, **characterised in that** stitch loops (22, 22') adjoining one another in circumferential direction are each formed by two filaments (23, 23') and the stitch loops adjacent axially and in circumferential direction are each formed by one of these filaments and by a respective other filament, wherein - in axial direction from stitch loop to stitch loop - the filaments have a part in the formation of the stitch loops respectively adjacent in circumferential direction and extend progressively in staircase-like manner around the endoprosthesis formed as a stent.

10. Endoprosthesis according to claim 4, **characterised in that** in each instance a filament (23") in the connecting regions (25') extends untwisted in the longitudinal direction of the prosthesis and that the other filament (24") is spirally wound around this filament (23").

11. Endoprosthesis according to one of claims 4 to 10, **characterised by** a detenting by at least one retaining rod (30) which extends in the longitudinal direction of the prosthesis and is anchored by one end thereof fixedly in the mesh and is provided at the other end with a hook (32) for detenting in a stitch loop.

12. Endoprosthesis according to claim 11, **characterised in that** the retaining rod (30) extends at the outer side along the hoselike mesh.

13. Endoprosthesis according to claim 11, **characterised in that** the retaining rod (30) extends through the stitch loop structure of the mesh in the manner of a warp thread.

14. Endoprosthesis according to one of claims 4 to 13, **characterised in that** for limitation of the length of the endoprosthesis formed as a stent the hoselike mesh is equipped with warp threads extending in the stent longitudinal direction.

15. Endoprosthesis according to claim 14, **characterised in that** the warp threads are connected at least in the region of the prosthesis ends with the filaments forming the stitch loops.

16. Endoprosthesis according to claim 14 or 15, **characterised in that** the warp threads consist of textile filaments and are arranged closely adjacent one another in such a manner that they form a casing encloses the stent.

17. Endoprosthesis according to one of claims 14 to 16, **characterised in that** the warp threads consist of biologically degradable material.

18. Endoprosthesis according to claim 17, **characterised in that** the warp threads are formed as medication depots and, when they degrade, release medications.

19. Endoprosthesis according to one of claims 14 to 16, **characterised in that** the warp threads consist of extensible material, such as texturised textile threads.

20. Endoprosthesis according to 14 or 15, **characterised in that** the warp threads consist of non-elastic material of high density and proton count.

21. Endoprosthesis according to one of claims 1 to 20, **characterised in that** at the end faces of the hoselike mesh the ends of the filaments are bent over and are connected with the mesh, for example interwoven, glued, soldered or welded.

22. Endoprosthesis according to claim 21, **characterised in that** the ends of the filaments at the end faces of the hoselike mesh are formed as eyes.

23. Endoprosthesis according to one of claims 1 to 22, **characterised in that** the filaments forming the stitch loops of the hoselike mesh are formed on the side facing the lumen to be rounded off or flattened off.

24. Endoprosthesis according to one of claims 1 to 23, **characterised in that** the filaments forming the mesh consist of super-elastic material.

25. Endoprosthesis according to one of claims 1 to 24, **characterised in that** the filaments forming the mesh consist of a memory material, for example of nitinol.

## Revendications

1. Endoprothèse (10) implantable par voie percutanée au moyen d'un cathéter dans le corps d'un patient, en particulier dans des vaisseaux ou organes tubulaires, laquelle est conçue sous forme de corps creux allongé et - après positionnement au bon emplacement pendant l'implantation - peut varier entre une petite lumière au cours de l'introduction et une lumière plus grande correspondant à la position d'utilisation, l'endoprothèse (10) étant formée par un treillis (11) tubulaire réalisé en filaments élastiques (13, 14), qui possède la structure d'un grillage en fil métallique avec des mailles (12) formant des polygones, dans lequel les filaments des mailles successives dans le sens de l'axe longitudinal de la prothèse s'enlacent de manière lâche dans les angles (15) desdites mailles, et les filaments (13, 14) étant munis, au niveau des angles (15) des mailles (12), de moyens d'ancrage (17, 18) entrant en prise par conjugaison de forme dans la position étirée de la prothèse, **caractérisée en ce que** les filaments de chacune des mailles successives dans le sens de l'axe longitudinal de la prothèse s'enlacent de manière lâche, de telle sorte que les mailles poussées les unes contre les autres entraînent un rétrécissement de la prothèse par rapport à sa longueur maximale, dans laquelle les filaments sont en contact l'un avec l'autre dans les angles (15) des mailles, et **en ce que** les moyens d'ancrage sont des creux (17, 18), qui sont formés par la compression des filaments (13, 14), se croisant dans les angles (15) dans la position étirée de la prothèse, jusqu'à obtenir pratiquement l'épaisseur d'un filament et qui entrent en prise dans la position allongée de la prothèse.

2. Endoprothèse selon la revendication 1, **caractérisée en ce que** le treillis tubulaire (11) est formé par au moins un filament (13, 14) continu et tressé en rond en formant des mailles.

3. Endoprothèse selon la revendication 1, **caractérisée en ce qu'**un treillis plat à l'origine est enroulé sous forme de tube et, à cet effet, deux bords longitudinaux du treillis sont assemblés par une couture longitudinale.

4. Endoprothèse (10) implantable par voie percutanée au moyen d'un cathéter dans le corps d'un patient, en particulier dans des vaisseaux ou organes tubulaires, laquelle est conçue sous forme de corps creux allongé et - après positionnement au bon emplacement pendant l'implantation - peut varier entre une petite lumière au cours de l'introduction et une lumière plus grande correspondant à la position d'utilisation, l'endoprothèse (10) étant formée par un treillis (21, 41) tubulaire en filaments élastiques (13, 14), qui possède la structure d'un grillage en fil métallique avec des mailles (22, 22', 36) formant des polygones, dans lequel les mailles comportent des zones de liaison formées par deux filaments torsadés l'un avec l'autre, **caractérisée en ce que** les filaments (23, 24 ; 23', 24'), dans les zones de liaison (25) des mailles contiguës dans le sens périphérique, sont torsadés l'un avec l'autre avec au moins une spire, et **en ce que** les filaments (23, 24 ; 23', 24'), par compression dans les zones de liaison torsadées, comportent des creux entrant en prise par conjugaison de forme à l'état dilaté, et les moyens d'ancrage formés par les torsades (27, 27') pouvant se désolidariser sous l'effet de forces de traction axiales et, à cet effet, par la perte de sa force radiale, la prothèse subit un allongement, ainsi qu'une variation de section vers une plus petite lumière.

5. Endoprothèse selon la revendication 4, **caractérisée en ce que** les zones de liaison (25) avec deux filaments (33, 34) torsadés l'un avec l'autre s'étendent dans le sens longitudinal de la prothèse.

6. Endoprothèse selon la revendication 4, **caractérisée en ce que** les zones de liaison (35, 35') formées par deux filaments (33, 34) torsadés l'un avec l'autre s'étendent le long de lignes - imaginaires-hélicoïdales d'une paroi cylindrique - également imaginaire - déployée par la prothèse et forment une structure hélicoïdale.

7. Endoprothèse selon la revendication 6, **caractérisée en ce que** la structure hélicoïdale est interrompue par la variation de direction des zones de liaison (35, 35') des mailles successives, de telle sorte que dans une partie des mailles, les zones de liaison (35') qui s'étendent entre celles-ci suivent un trajet formant un angle avec les zones de liaison (35) entre d'autres mailles.

8. Endoprothèse selon une des revendications 4 à 6, **caractérisée en ce que** des mailles (22, 36) contiguës dans le sens périphérique sont formées chacune par deux filaments (23, 24 ; 33, 34) et des mailles (22, 36) contiguës dans le sens axial et assemblées par une zone de liaison (25, 35) sont formées chacune par les deux mêmes filaments.

9. Endoprothèse selon une des revendications 4 à 6, **caractérisée en ce que** des mailles (22, 22') contiguës dans le sens périphérique sont formées chacune par deux filaments et les mailles contiguës dans le sens axial et dans le sens périphérique sont formées chacune par l'un de ces filaments et par un autre filament, moyennant quoi, dans le sens axial de maille en maille, les filaments contribuent à la formation des mailles contiguës dans le sens périphérique et s'étendent progressivement en forme d'escaliers autour de l'endoprothèse conçue sous forme de stent.

10. Endoprothèse selon la revendication 4, **caractérisée en ce qu'**un filament (23'') s'étend dans les zones de liaison (25') à l'état non torsadé dans le sens longitudinal de la prothèse et **en ce que** l'autre filament (24'') est enroulé en forme de spirale autour de ce filament (23'').

11. Endoprothèse selon une des revendications 4 à 10, **caractérisée par** un moyen d'ancrage formé par au moins une tige de support (30), qui s'étend dans le sens longitudinal de la prothèse et dont une des extrémités est ancrée solidement dans le treillis et l'autre extrémité est munie d'un crochet (32) destiné à être ancré dans une maille.

12. Endoprothèse selon la revendication 11, **caractérisée en ce que** la tige de support (30) s'étend sur le côté extérieur le long du treillis tubulaire.

13. Endoprothèse selon la revendication 11, **caractérisée en ce que** la tige de support (30) s'étend à la manière d'un fil de chaîne à travers le réseau de mailles du treillis.

14. Endoprothèse selon une des revendications 4 à 13, **caractérisée en ce que** pour limiter la longueur de l'endoprothèse conçue sous forme de stent, le treillis tubulaire est muni de fils de chaîne qui s'étendent dans le sens longitudinal du stent.

15. Endoprothèse selon la revendication 14, **caractérisée en ce que** les fils de chaîne, au moins dans la zone des extrémités de la prothèse, sont assemblés avec les filaments formant les mailles.

16. Endoprothèse selon la revendication 14 ou 15, **caractérisée en ce que** les fils de chaîne sont formés par des filaments textiles et sont disposés étroitement les uns contre les autres, de telle sorte qu'ils forment une gaine entourant le stent.

17. Endoprothèse selon une des revendications 14 à 16, **caractérisée en ce que** les fils de chaîne sont formés par un matériau biodégradable.

18. Endoprothèse selon la revendication 17, **caractérisée en ce que** les fils de chaîne sont conçus sous forme de dépôts de médicaments et libèrent les médicaments au moment de leur dégradation.

19. Endoprothèse selon une des revendications 14 à 16, **caractérisée en ce que** les fils de chaîne sont formés par un matériau extensible, tels que des fils textiles texturés.

20. Endoprothèse selon une des revendications 14 à 16, **caractérisée en ce que** les fils de chaîne sont réalisés dans un matériau non élastique à haute densité et avec un indice de protons élevé.

21. Endoprothèse selon une des revendications 1 à 20, **caractérisée en ce que** les extrémités des filaments sont coudées au niveau des extrémités frontales du treillis tubulaire et sont assemblées avec le treillis par tressage, collage, brasage ou soudage.

22. Endoprothèse selon la revendication 21, **caractérisée en ce que** les extrémités coudées des filaments au niveau des extrémités frontales du treillis tubulaire sont conçues sous forme d'oeillets.

23. Endoprothèse selon une des revendications 1 à 22, **caractérisée en ce que** les filaments formant les mailles du treillis tubulaire sont arrondis ou aplatis sur le côté orienté vers la lumière.

24. Endoprothèse selon une des revendications 1 à 23, **caractérisée en ce que** les filaments formant le treillis sont réalisés dans un matériau superélastique.

25. Endoprothèse selon une des revendications 1 à 24, **caractérisée en ce que** les filaments formant le treillis sont réalisés dans un matériau à mémoire de forme, tel que le nitinol.
